# EUROPEAN PATENT SPECIFICATION

(11) **EP 3 355 285 B1**
(45) Date of publication and mention of the grant of the patent: **04.09.2019**
(21) Application number: 17154059.4
(22) Date of filing: 31.01.2017
(51) Int. Cl.: G07F 11/00

(54) **STERILISING AUTOMATIC DISPENSER OF DISPOSABLE DEVICES FOR AID OF FEMALE URINATION IN A STANDING POSITION**
AUTOMATISCH STERILISIERENDER VERKAUFSAUTOMAT FÜR VORRICHTUNGEN ZUR UNTERSTÜTZUNG DES URINIERENS VON FRAUEN IN STEHENDER POSITION
STÉRILISATION DE DISTRIBUTEUR AUTOMATIQUE DE DISPOSITIFS JETABLES POUR AIDER À LA MICTION DES FEMMES EN POSITION DEBOUT

(43) Date of publication of application: 01.08.2018
(73) Proprietor: Saniplast S.r.l., 20123 Milano (IT)
(72) Inventor: MERRY DEL VAL, Domingo, 20123 Milano (IT)
(74) Representative: Petruzziello, Aldo

(56) References cited:
- WO-A1-93/11691
- DE-U1-212006 000 067
- US-A- 5 335 822
- US-A- 5 941 414
- US-A- 6 098 840
- US-B1- 7 694 873

## Description

The present invention relates to an automatic sterilising dispenser of disposable devices for aid of female urination in the standing position ("Feminine Urinary Device" - FUD).

The term "Feminine Urinary Device" (FUD) is used to identify support devices that allow women to urinate in a standing posture in situations where this is necessary for hygienic reasons, for example when using public bathrooms, or when for reasons of work or sport typically male clothes (trousers, jumpsuits, etc.) are worn.

These devices can be of various shapes, sizes and materials, rigid or flexible, reusable or disposable.

Currently these devices are sold online or in specific stores, in appropriate packaging which ensures sterility thereof.

One of these devices, of the disposable type, was the object of a European patent application EP 594253 A1 in the name of the Applicant.

The device described in EP 594253 A1 consists of a channel element, of substantially conical shape, which comprises a wide end with indentation, which is tilted with respect to the axis of the cone, said wide end acting as a larger base, and an outlet end with oblique opening, opposite to the larger base, which acts as a spout.

This disposable element is made in flexible and foldable material, such as waterproof paper, adequately stiffened, which appears flattened on the two opposite generatrices of the cone in order to be opened in a tubular manner at the time of use.

Since, as is known, the queues in women's toilets are much longer than those in men's toilets, because of the need of having to partially undress and then get dressed again, the advantage of using such "Feminine Urinary Devices" (FUDs) in said toilets accessible to the public is clear. However, at present, this is only possible if one has previously equipped oneself with these devices as there are no automatic dispensers of these devices installed in women's toilets.

The advantage of having available automatic dispensers of sterile "Feminine Urinary Devices" (FUDs), installable in women's public toilets, preferably automatic dispensers small in size, is clear.

Document WO93/11691 discloses a device which has a first end with an opening for receiving urine from a female, the opening having a circumference which is contoured to fit around the outside of the female vulva. A second end opposite the first end is also provided with an opening. The second end opening allows urine to be discharged from the device. First and second longitudinal creases extend from the first end of the device to the second end and define first and second walls.

The patent US 6098840 describes an apparatus for dispensing credit cards, phone cards or the like, regular in shape and very slim, which however is not suitable for dispensing products that are not flat and irregularly shaped such as for example FUDs.

The patent US 5941414 describes an apparatus for dispensing slim products with a regular and flat shape, such as tickets, cards and the like, with a pull mechanism which is not suitable for dispensing the FUD in that the latter, as already mentioned, is not a flat product, and does not have a regular shape as a leaf or sheet, essential for handling with this equipment.

The patent US 5857588 also describes an apparatus for dispensing tickets, cards and the like, also of different thickness, which are dispensed through falling through a tilted pan, in which, however, the push mechanism is only suitable for parallelepiped-shaped objects, similarly to previous patents.

The patent US 5335822 also discloses an apparatus similar to that described in US 5857588.

The object of the present invention is to overcome, at least in part, the disadvantages of the prior art by providing a system capable of making available in women's toilets accessible to the public, for example in motorway restaurants, the abovementioned sterile FUD devices, in particular those with minimum overall dimensions, such as for example those that appear flattened and without packaging, so as to contain the size of said system.

Another object is to provide such a system that is also able to dispense sterile yet disposable FUD devices so as to avoid possible contaminations resulting from a possible insufficient cleaning of the reusable device.

Yet another object of the present invention is that of realising such a dispensing device able to supply, at a low price, an FUD of irregular shape and not flat, without packaging but perfectly intact and clean, as close as possible to sterility, avoiding contamination of it during handling.

These and other objects are achieved by the automatic sterilising dispenser in accordance with the invention having the features listed in the appended independent claim.

Advantageous embodiments of the invention are disclosed by the dependent claims.

An object of the present invention relates to an automatic dispenser apt to deliver a disposable "Feminine Urinary Device" (FUD), of flattened shape with an indentation and devoid of a packaging pack, characterised in that it is provided with a system of sanitising and/or sterilising elements, such as for example UV lamps, placed at least at the product outlet slot so as to dispense a disposable FUD device which is sterilised at the time of withdrawal and therefore shortly before use.

This sterilising automatic dispenser is a vending device intended, preferably, to be installed directly in the women's toilets of workplaces, service stations, department stores, cinemas, theatres and other public places where toilets are provided.

Said automatic dispenser can be a wall dispenser or in the form of an automatic or self-service machine, without thereby departing from the scope of the present invention.

Furthermore, this automatic dispenser can dispense the product free of charge or against payment, without thereby departing from the scope of the present invention.

In particular, said automatic dispenser has a system of forward movement and dispensing of FUDs specifically designed to dispense the disposable FUD device with indentation, distributed in a flattened form and without packaging (loose), in particular described in the European patent application EP 594253 A1 in the name of the Applicant, incorporated herein in full by reference.

As mentioned, such a device aiding female urination in the standing position ("Feminine Urinary Device" - FUD), which is suitable for being dispensed by the present automatic device, consists of a channel element of substantially conical shape, which is able to be flattened on the two opposite generatrices of the cone in order to be dispensed easily and then opened in a tubular manner at the time of use.

Said FUD comprises:
- a wide end with an indentation with curvilinear profile, said end which is tilted with respect to the axis of the cone, acting as a larger oblique base, and
- an oblique outlet end (smaller base), opposite to the larger base and provided with aperture, which serves as a spout.

This disposable element is made in a flexible and foldable material, such as waterproof paper, adequately stiffened, which appears flattened on the two opposite generatrices of the cone in order to be easily dispensed individually and then opened in a tubular manner at the time of use.

The system of forward movement and dispensing of FUDs provides for the forming of a longitudinal slot on the horizontal base on which the FUD to be dispensed rests and the positioning of the specific forward movement and pushing means below said slot so as to allow the direct contact of said FUD with said forward movement means, in particular the direct contact between said indentation and said forward movement and dispensing means, in such a way as to translate horizontally said FUD, resting on said base, towards the dispensing slot so as to make exit therefrom a section of FUD suitable for efficient gripping by the user.

In the present description the terms "product", "product to be dispensed" will be used interchangeably as synonyms of FUD ("Feminine Urinary Device").

It should be noted that the dispensing of a single product without packaging allows the supply of an FUD at an affordable price: in fact the known devices aiding female urination in a standing position are dispensed with a packaging in which many units of said devices are generally contained, and therefore not suitable for supply in toilets.

Moreover, the absence of packaging, for example of box or package type, makes it impossible to apply the known pusher systems, for example the spiral ones, to the present FUD, also in light of the fact that it has a large curvilinear indentation which is difficult to grip and push by the known dispensing means.

Further features of the invention will be made clearer by the following detailed description, referred to one of its purely non-limiting examples, illustrated in the accompanying drawings in which:
Figure 1 is a schematic perspective view of the automatic dispenser in accordance with the invention, in its closed configuration in use;
Figure 2 is a schematic perspective view of the automatic dispenser of Fig. 1 in accordance with the invention, in its open configuration and not in use, which houses a push dispensing and forward movement system according to a first embodiment;
Figure 3 is an enlarged perspective view of the back of the front door of the dispenser of Fig. 2, in its open configuration and not in use;
Figure 4 is a side vertical section of the dispenser of Fig. 1 in accordance with the invention;
Figure 5 is an enlarged perspective view of the forward movement and dispensing system of the embodiment illustrated in Figures 2-4;
Figure 6 is a plan and partial view of a horizontal section of the forward movement system taken along line VI-VI of Figure 4;
Figure 7 is an enlarged perspective view of the back of the front door, similar to Figure 3, of a variant of the forward movement system of the dispenser not in accordance with the invention;
Figure 8 is a partial side view of Fig. 7 illustrating the container for loading of the product of the dispenser, not according to the invention;
Figure 9 is an enlarged perspective view of the forward movement system variant shown in Figures 7 and 8, not according to the invention;
Figure 10 is a plan view of Figure 8.

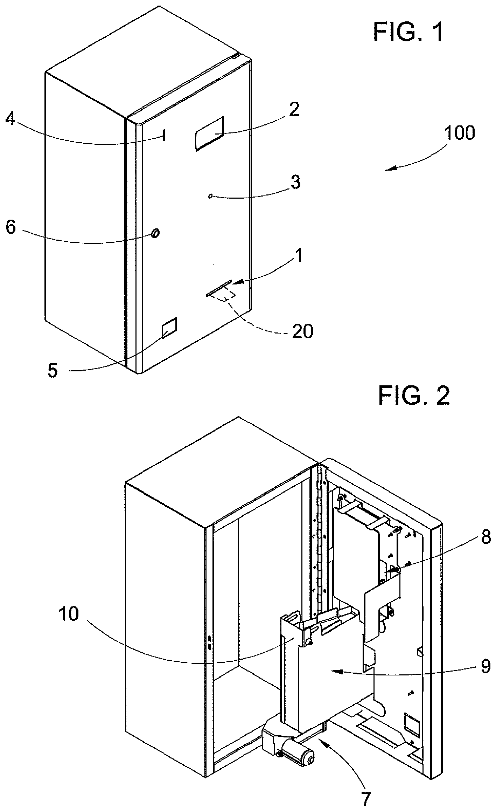

Figure 1 illustrates a sterilising automatic dispenser of flattened disposable devices as an aid to female urination in the standing position (normally identified with the name "Feminine Urinary Device" - FUD), denoted overall by reference numeral 100.

The automatic dispenser 100 is composed of an outer casing, generally of parallelepiped shape, although this shape is not binding for the purposes of the present invention.

Moreover also the dimensions of this casing are not binding for the purposes of the present invention, and can be, for example, 780 x 410 x 340 (mm), in the case of parallelepiped shape.

As mentioned, the automatic dispenser 100 can adopt, or otherwise, a payment system of the type known in the art, such as a coin system or change giver coin system for payment with coins or other conventional payments systems such as RFID card, banknote reader, etc., without thereby departing from the scope of the present invention.

Figure 1 shows a coin payment system even if this is not binding for the purposes of the present invention.

On the outer face of the front door of this automatic dispenser 100 the following components are present:
- a slot 1 for the output of the dispensed product (FUD indicated as a dotted line in Figure 1 by reference numeral 20;
- a display 2 (optional), possibly of the touch-screen type, to display the cost and/or the operating instructions for the user and/or operations that guide the user during purchase/withdrawal of the product;
- at least one button 3, possibly shown on the display 2 if of the touch-screen type, although there may be several buttons depending on the complexity of operation and of management of the various optional components;
- a slot 4 for the insertion of coins (optional);
- a window 5 for the withdrawal of the change (optional).
In addition, on this outer face a key lock 6 can be provided for closing/opening said front door, even if this lock can be provided on the side wall of the dispenser 100 without thereby departing from the scope of the present invention.

Moreover, on said outer face of said front door a warning light can also be present (optional and therefore not illustrated) to indicate the sold-out product.

As illustrated in Figures 2-3, behind the front door, and attached thereto, are the electronic management components 8, for example in the form of electronic card, needed to manage all the components present and the handling/forward movement of the FUD as will be described in detail here below.

Also attached to the back of said front door, a loading container 9 is placed for housing a stack of said flattened disposable FUD devices, preferably the FUD devices described in the European patent application EP 594253 in the name of the Applicant, provided with an indentation.

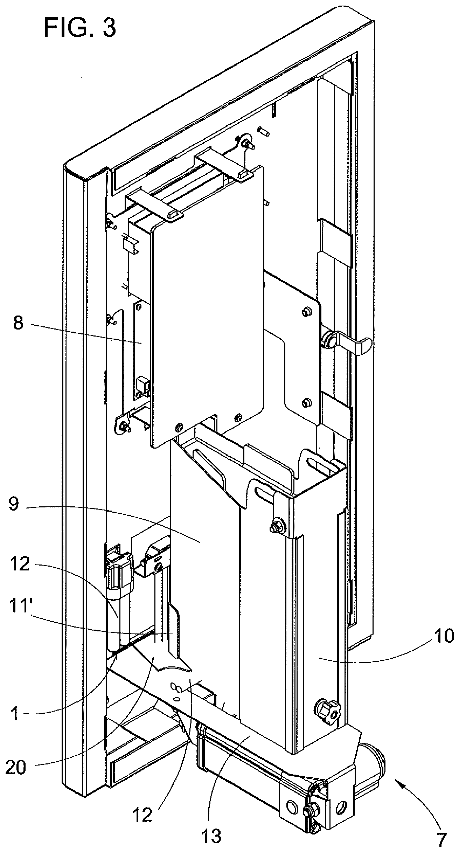

Said loading container 9 is composed of a metal container, preferably of shape suitable for containing the flattened FUD described in EP594253, generally open on the bottom, which rests on a horizontal plate 13 (Fig. 3) extending as far as the outlet slot 1 so that said plate 13 is aligned with said outlet slot 1, and substantially at the same height of said outlet slot 1.

It is understood that the plate 13 which acts as a lower base for the container 9 may be integral with the container 9 without thereby departing from the scope of the present invention.

Below the support plate 13 or base a push forward movement and dispensing system 7 is placed which will be explained in detail here below.

Said loading container 9 is further provided, preferably, with a closure/opening element 10, in the form of a front door so as to allow a good visual inspection of the state of the stack, avoiding at the same any loading from above.

Furthermore, the container 9 may be provided with a possible sensor for the indication of sold-out product or stock at a minimum (not shown in the drawing).

Having loaded the container 9 with a stack of flattened FUD devices, the insertion is provided on the upper part of the stack of FUDs of a shaped counterweight (not shown in the drawings) to keep compact the stack of the product to be dispensed and maintain the FUD on the bottom of the container 9, always in contact with the plate 13, facilitating the dispensing thereof as will be explained in detail here below.

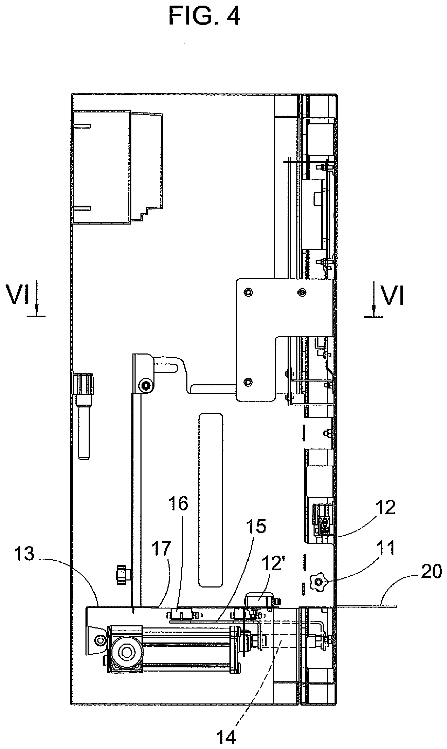

Also provided on said container 9, on the opposite wall to the door 10, a door 11' adjustable in height thanks to two adjustment screws 11 (Figure 4) that allow this door 11' to slide vertically along the walls of the container 9 so as to be able to fix appropriately the height of the outlet slot of the FUD 20 from the container 9 depending on the thickness and type of FUD loaded in the container 9.

In the drawings accompanying the present application, the stack of FUDs 20 is placed in the container 9 with the long side of the FUD in contact with the long side of the container 9, in turn aligned with the sides of the dispenser 100 so as to make exit the short side of the FUD 20 from the slot 1: however it is understood that the FUD 20 can exit from the long side, without thereby departing from the scope of the present invention.

Behind the front door there are also, in the vicinity of the outlet slot 1, one or more UV lamps 12 to abate physical and chemical contaminants, viruses, bacteria and moulds, mites, pollens, etc. which are among the main causes of diseases for humans.

Although each of these contaminants is dispersed in a different way, the actual circulation of pollutants in the air means that they can be introduced and multiply.

The use of UV-C has been included among the best practices for protecting objects against contaminations of all kinds.

Ultraviolet rays are electromagnetic waves and are divided according to the wavelengths, expressed in nanometres (nm). Ultraviolet waves with germicidal properties are those comprised between 100 and 280 nm. When bacteria, viruses and other micro-organisms are exposed to UV-C germicidal light with wavelength 253.7 nm, they are inactivated and can no longer pose a threat to human health.

The treatment with UV-C rays is purely physical, and highly ecological, allowing great effectiveness always to be obtained and enabling a considerable reduction in the level of microbial load, maintaining the shelf-life of the products intact, without any contraindication.

The UV-C rays germicidal lamps with direct radiation for the deep disinfection of air and surfaces installed in the present dispenser allow elimination of micro-organisms, including bacteria and moulds, obtaining a 99% reduction.

One or more UV lamps 12' can also be advantageously provided inside the container 9 (Fig. 3) so as to sterilise the entire length of the FUD that is moved towards the outlet slot 1 during dispensing, as will be described subsequently in detail.

Referring to Figures 4-6, the embodiment of the pusher system 7 will now be illustrated that enables the product to be dispensed to be moved forwards in the slot 1 for withdrawal provided by means of a direct push on the curved portion, otherwise here also referred to as indentation, of said FUD 20 as will be illustrated in detail here below.

In said first embodiment, the pusher system 7, which as mentioned is placed below the plate 13 for support of the loading container 9 (Fig. 3), is an electromechanical linear actuator.

The linear actuator is a mechanism that converts the rotary motion of an electric motor into a linear movement allowing loads to be pushed or pulled.

The present linear actuator 7 is composed of a drive motor with reduction unit, and an actuator stem 14 that moves linearly toward the outlet slot 1 and from said outlet slot 1 towards the interior of the dispenser 100.

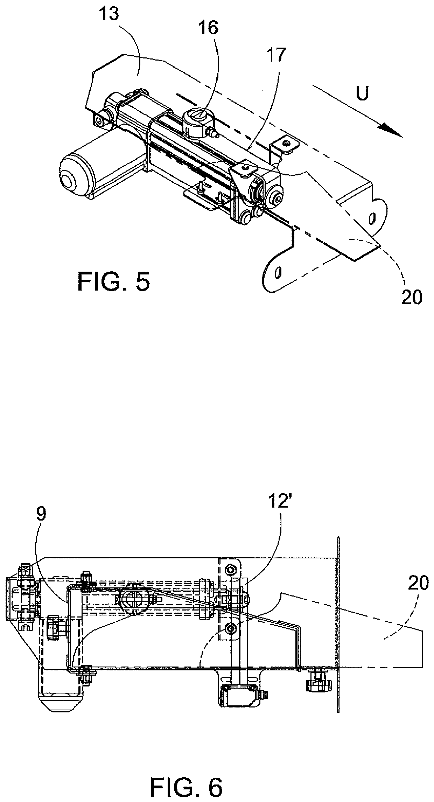

Mounted on said actuator stem 14, at the free end, is a rod or arm 15, L-shaped, which in turn has mounted at its free end a mechanical contrast element 16, or ejection block: in this way a linear movement of the stem 14 towards the outlet slot 1 corresponds to a linear movement of the block 16 also towards the outlet slot 1 (indicated by the arrow U in Figure 5).

Said block 16 slides forwards and backwards inside a longitudinal aperture 17 (Fig. 5) formed in a longitudinal portion of the plate 13 whereon the stack of FUDs 20 contained in the container 9 rests.

The aperture 17, with an elongated shape, is in fact formed at a zone of the plate 13 whereon at least part of the recess (indentation) of the FUD 20 will come to rest.

The length of this longitudinal aperture or slot 17 is such as to provide the FUD 20 with a sufficient movement to make a predefined portion of the FUD 20 exit from the outlet slot 1.

Said block 16, normally in the form of a small cylinder, has a cylindrical head with a portion thereof which projects partially upwards from the slot 17 with respect to the remaining part of the head (portion not projecting upwards).

Said projecting portion (or protuberance) of said block 16 advantageously has, at its front side (the one frontal to the outlet slot 1), a curved profile that is complementary to the profile of the indentation of the FUD as shown in Figure 6: in this way said curved projecting portion of said block 16 can act as a pusher of the FUD 20 when the stem 14 moves forwards in the direction of the arrow indicated by U in Figure 5, towards the outlet slot 1.

More particularly, when the stem 14 is fully retracted, the protruding part of the block 16 is also in abutment against a part of the inlet of the FUD 20 to be dispensed, which is placed above the slot 17: during the extension stroke of the stem 14 that makes the block 16 move forwards (in the direction of the arrow indicated by U in Figure 5), the abovementioned protuberance of the block 16 will thus push the FUD 20 forwards in the slot 1 of withdrawal where the user can easily pull it out as shown in Figure 6.

During the return stroke of the stem 14 (in the opposite direction to the arrow U in Fig. 5), instead, the non-projecting portion of the head of the block 16 will meet the FUD 20 first with respect to the protuberance of the block 16, without acting in any way on said FUD in that not in contact.

Therefore said projecting portion of said block 16 also acts as end of stroke or stop when said block 16 is retracted as a result of the retraction of the stem 14, once the FUD has been dispensed, since said projecting portion abuts upwards against the rear end of the slot 17 (Fig. 5).

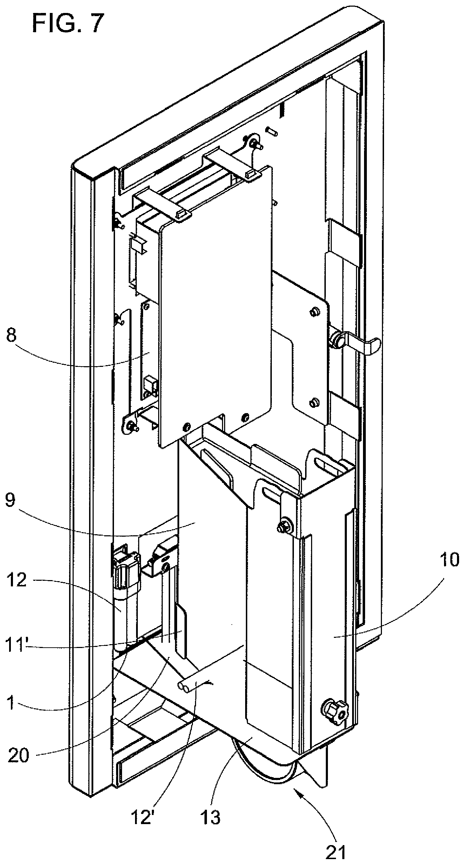

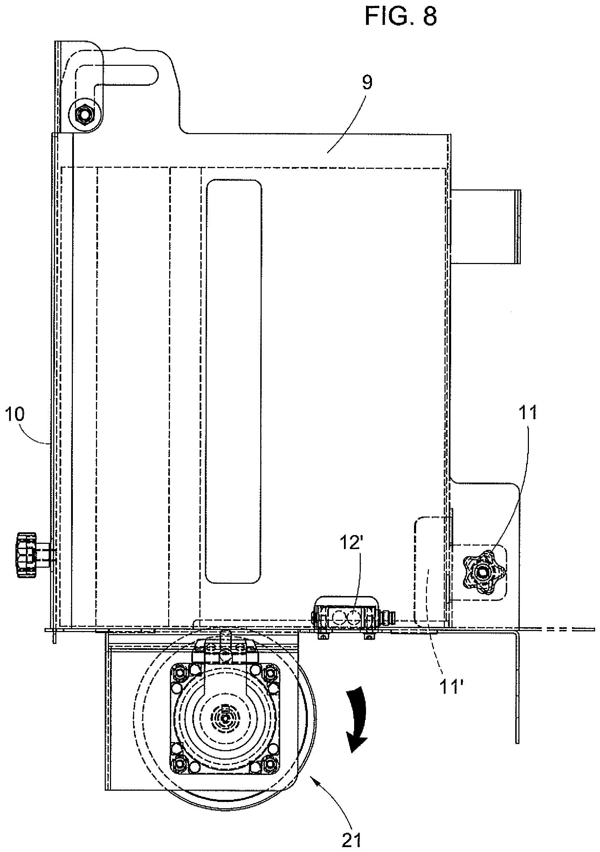

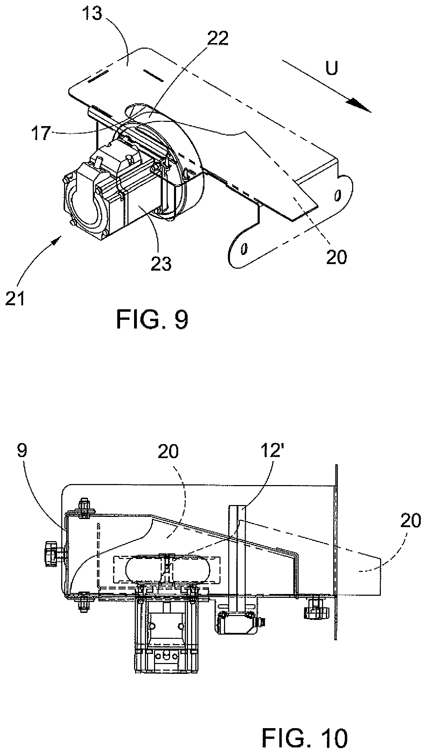

A variant of the pusher, not according to the invention, can be represented by a system with at least one free rubber-covered wheel illustrated in Figures 7-10.

With the exception of this second embodiment of the pusher system, the device 100 has the same elements and features of the previously described dispenser and therefore the same reference numerals will be used here to denote the same components shown in Figures 1-6.

More particularly, behind the front door shown in Figure 7, there are substantially the same components indicated in Figures 1-6: the electronic management components 8, the container 9 for loading of the product to be dispensed, the UV lamps 12 and 12' (inside the container 9), the plate 13 with the longitudinal aperture 17 on which the container 9 rests, with the exception of the pusher system 21 placed below the plate 13 in order to push appropriately the product to be dispensed in the appropriate slot 1 for withdrawal of the same by the user.

The pusher system 21, located below the plate 13 with slot 17, is formed essentially by at least one free rubber-covered wheel 22 attached to a motor or geared motor 23.

Said free rubber-covered wheel 22 which is motorised allows the product to be dispensed to be moved forwards in the slot 1 provided for withdrawal by means of contact with friction of said FUD 20 with said rubber-covered wheel 22.

The slot or longitudinal slit 17 is formed on the plate 13 at the area in which the stack of FUDs 20 rests so as to put into direct contact the surface of the rubber-covered wheel 22 with the surface of the FUD 20.

The length of this slot 17 is at least slightly greater with respect to the diameter of the rubber-covered wheel 22, even if this is not binding for the purposes of the present invention.

The surface of the rubber-covered wheel is preferably uneven so as to create a certain friction with the material that forms the FUD 20.

The wheel-FUD contact is also guaranteed by the counterweight (not shown) placed in the top of the stack of FUDs 20 as mentioned previously.

The dimensions of the wheel 22 are such as to impart to the FUD 20 a predetermined forward movement of such length as to allow the exiting of a portion of FUD sufficient in order to be able to comfortably extract said FUD 20 from the outlet slot 1.

Thanks to the rotary movement of the rubber-covered wheel 22, the FUD 20 is pushed forwards towards the outlet slot 1 for the withdrawal of the same by the user, similarly to what was described previously in relation to the linear actuator 7.

It is understood that other embodiments of pusher means may be provided, for example with two rubber-covered wheels connected one to the other by means of a belt, or using appropriate cams that by performing a semicircular motion dispense the product in a hygienic and convenient position for the user.

As mentioned, it is understood that the FUD product 20 can be dispensed from the long side rather than the short one as shown in the accompanying drawings, without thereby departing from the scope of the present invention.

Irrespective of the type of pusher used, inside the automatic dispenser 100 an electric panel (not shown) is also provided with all the electrical components for the operation of the same.

In relation to the automatic management of the dispensing process, the present automatic dispenser 100 provides, as mentioned, at least one electronic power board with the function of controlling all I/O components of the dispenser 100 and managing the electric actuators or motors, constantly dialoguing with the management software.

However in the case of a display 2 of the touch-screen type it is preferable to use two electronic cards:
- the first one formed by a power board that deals with the entire control and management of the product dispensing cycles in which the entire sensor part of input (IN) is housed together with the entire power actuation part (OUT);
- the second one formed by a surveillance card for the interface that deals with providing all the interface tools needed by the user using a TFT system with touch screen.

The touch-screen display 2 has the function of interfacing with the user as well as with the personnel carrying out maintenance on the dispenser. This monitor gives all the instructions, including graphic ones, to facilitate the operation of dispensing of the product by the end user.

The various service menus are also given which are useful to the maintenance worker in order to set the parameters of the machine, as well as perform the working tests of the various internal components of the same, download the sales listed and carry out any of the dispenser software updates.

This display has the particular feature of being able to use the following types of connections: Ethernet, modem (optional) and Wi-Fi (optional). These connections allow management and control of the dispenser in an advanced manner, for example by means of specially created Apps that can be installed on smartphones or palmtops which interface directly with the dispenser to obtain sales data, or anything else that might be considered important. The control of the dispenser can also be performed via the Web interface (optional).

If there is a coin system with systems for giving change, it can be composed of an inlet duct, the central part where the store of coins is present and the change giver with its channel bringing the coins into the appropriate space.

The present FUD dispenser is therefore a specific dispensing machine for dispensing individual FUDs without packaging at a very low price, which can be installed in all public women's toilets (railway stations, motorway restaurants, stadiums, theatres, etc.), which is not feasible with any of the dispensing machines existing to date, much less so with the machines described in the prior art patents mentioned above: proof of this is the fact that none of the similar products that are on the market for decades has been able to use this form of sale.

The present invention is not limited to the particular embodiments described previously and illustrated in the accompanying drawings, but instead numerous detail changes may be made thereto within the reach of the person skilled in the art, without thereby departing from the scope of the same invention, as defined in the appended claims.

## Claims

1. Automatic sterilising dispenser (100) of single disposable devices (20) as an aid for female urination in a standing position ("Feminine Urinary Device" - FUD), each FUD (20) being made up of a substantially conical channel element which is substantially flattened on the two opposite generatrices of the cone in order to be dispensed and subsequently opened in a tubular manner at the time of use,
said FUD (20) having a larger oblique base with a curvilinear profile and provided with an indentation, and a smaller oblique base, opposite the larger base, provided with an opening forming a spout,
said automatic dispenser (100) comprising
- a stack of said single disposable FUD devices in substantially compacted form (20);
- at least one loading container (9) housing said stack of said single disposable FUD devices in substantially compacted form (20), said container (9) resting on a horizontal base (13);
- an outlet slot (1) dispensing said FUD (20), in the vicinity of which one or more UV steriliser elements (12) are placed, said slot (1) being aligned with said base (13);
- a dispensing button (3);
- at least one electronic component (8) for dispensing control;
- means of forward movement and dispensing (7) placed beneath said base (13) of said loading container (9) and a slot (17) formed through said base (13),
wherein said forward movement means (7) are apt to move the FUD (20) resting on said base (13) forwards for a certain length toward said outlet slot (1), by means of direct push on said indentation of said FUD (20) in such a way as to cause to exit from said outlet slot (1) a certain length of FUD for gripping by the user,
said forward movement means (7) comprising an element which acts as mechanical contrast (16) moving along said horizontal slot (17) and having a part protruding upwards from said slot (17), said protruding part having a curved profile, at the frontal side to the outlet slot (1), that is complementary to the profile of the indentation of the FUD so as to exert a direct push on at least one curved portion of said indentation of said FUD (20) when placed above said slot (17) of said base (13).

2. Device (100) according to claim 1, further provided with a coin payment system or with other payment systems, such as RFID card, banknotes reader, and the like.

3. Device (100) according to claim 2, further provided with a display (2), possibly of the touch-screen type, for displaying the cost and/or the instructions for use for the user and/or the operations guiding the user during the purchase/withdrawal of the product.

4. Device (100) according to any one of the preceding claims, wherein said container (9) provides a sensor for indicating sold-out product or minimum stock, connected to a warning light placed on the outer side of said device.

5. Device (100) according to any one of the preceding claims, wherein said container (9) is further provided with an opening/closure element (10) for the front loading of said stack of FUDs (20), and/or with a shaped counterweight for maintaining said stacked FUDs (20) compacted.

6. Device (100) according to any one of the preceding claims, wherein said container (9) provides one or more UV lamps (12') so as to sterilise the FUD (20) moving forwards towards the outlet slot (1) during the dispensing.

7. Device (100) according to any one of the preceding claims, wherein said means (7) of forwarding movement by direct push are in the form of an electromechanical linear actuator comprising:
- a drive motor with reduction unit, and
- an actuator stem (14) having mounted at its free end an arm (15) which, in turn, has mounted, at its free end, said mechanical contrast element (16) with head partially projecting from said slot (17) and sliding along it, so as to push at least one portion of said indentation of said FUD (20) which is placed above said slot (17) of said base (13).

8. Device (100) according to any one of the preceding claims, wherein said outlet slot (1) of said FUD (20) and said horizontal base (13) are aligned and both placed at the same height.

9. Device (100) according to any one of the preceding claims, comprising moreover one or more of the following components:
- an electrical panel for the operation of the electrical components;
- at least one power board (8) for controlling and managing the product dispensing cycles;
- an optional surveillance card for interfacing with a touch-screen display (2).

## Patentansprüche

1. Automatisch sterilisierender Spender (100) von einzelnen wegwerfbaren Vorrichtungen (20) als Hilfsmittel für das weibliche Urinieren im Stehen ("Feminine Urinary Dispenser" - FUD), wobei jedes FUD (20) aus einem im Wesentlichen konischen Kanalelement besteht, das auf den beiden gegenüberliegenden Mantellinien des Kegels im Wesentlichen abgeflacht wird, um zum Zeitpunkt der Verwendung abgegeben und anschließend rohrförmig geöffnet zu werden,
wobei das FUD (20) eine größere schräge Grundfläche mit einem gekrümmten Profil aufweist und mit einer Vertiefung versehen ist, und eine kleinere schräge Grundfläche, die der größeren Grundfläche gegenüberliegt und mit einer Öffnung versehen ist, die einen Auslauf bildet,
wobei der automatische Spender (100) umfasst
- einen Stapel der einzelnen wegwerfbaren FUD-Vorrichtungen in im Wesentlichen kompaktierter Form (20);
- mindestens einen Ladebehälter (9), der den Stapel der einzelnen wegwerfbaren FUD-Vorrichtungen in im Wesentlichen kompaktierter Form (20) aufnimmt, wobei der Behälter (9) auf einer horizontalen Basis (13) ruht;
- einen Ausgabeschlitz (1), der die FUD (20) abgibt, in dessen Nähe ein oder mehrere UV-Sterilisationselemente (12) angeordnet sind, wobei der Schlitz (1) mit der Basis (13) ausgerichtet ist;
- einen Ausgabeknopf (3);
- mindestens eine elektronische Komponente (8) zur Ausgabesteuerung;
- Mittel zur Vorwärtsbewegung und Ausgabe (7), die unterhalb der Basis (13) des Ladebehälters (9) angeordnet sind, und einen Schlitz (17), der durch die Basis (13) hindurch ausgebildet ist,
wobei die Vorwärtsbewegungsmittel (7) geeignet sind, das auf der Basis (13) aufliegende FUD (20) um eine bestimmte Länge nach vorne zu dem Ausgabeschlitz (1) zu bewegen, und zwar durch direkten Druck auf die Vertiefung des FUD (20), sodass aus dem Ausgabeschlitz (1) eine bestimmte Länge des FUD zum Greifen durch den Benutzer austreten kann,
wobei die Vorwärtsbewegungsmittel (7) ein Element umfassen, das als horizontaler mechanischer Kontrast (16) wirkt, der sich entlang des horizontalen Schlitzes (17) bewegt und einen Teil aufweist, der nach oben aus dem Schlitz (17) herausragt, wobei der herausstehende Teil ein gekrümmtes Profil an der Vorderseite des Ausgabeschlitzes (1) aufweist, das zu dem Profil der Vertiefung des FUDs komplementär ist, um einen direkten Druck auf mindestens einen gekrümmten Abschnitt der Vertiefung des FUDs (20) auszuüben, wenn es über dem Schlitz (17) der Basis (13) angeordnet ist.

2. Vorrichtung (100) nach Anspruch 1, ferner versehen mit einem Münzzahlungssystem oder mit anderen Zahlungssystemen, wie RFID-Karte, Banknotenleser und dergleichen.

3. Vorrichtung (100) nach Anspruch 2, die ferner mit einem Display (2), möglicherweise vom Typ eines Touchscreens, das zum Anzeigen der Kosten und / oder der Gebrauchsanweisung für den Benutzer und / oder zum Anleiten des Benutzers während dem Kauf / der Entnahme des Produkts vorgesehen ist.

4. Vorrichtung (100) nach einem der vorhergehenden Ansprüche, wobei der Behälter (9) einen Sensor zum Anzeigen eines ausverkauften Produkts oder eines Mindestbestandes aufweist, der mit einer Warnleuchte auf der Außenseite der Vorrichtung verbunden ist.

5. Vorrichtung (100) nach einem der vorhergehenden Ansprüche, wobei der Behälter (9) ferner mit einem Öffnungs-/Verschlusselement (10) für die Frontbeladung des FUD- Stapels (20) und / oder mit einem geformten Gegengewicht zum Kompaktieren der gestapelten FUDs (20) versehen ist.

6. Vorrichtung (100) nach einem der vorhergehenden Ansprüche, wobei der Behälter (9) eine oder mehrere UV-Lampen (12') aufweist, um die FUD (20) zu sterilisieren, die sich während der Ausgabe vorwärts in Richtung des Auslassschlitzes (1) bewegen.

7. Vorrichtung (100) nach einem der vorhergehenden Ansprüche, wobei die Mittel (7) der Vorwärtsbewegung durch direkten Druck in Form eines elektromechanischen linearen Aktuators vorliegen, der umfasst:
- einen Antriebsmotor mit Untersetzungseinheit und
- eine Aktuatorstange (14), die an ihrem freien Ende einen Arm (15) montiert hat, der wiederum an seinem freien Ende das mechanische Kontrastelement (16) mit einem Kopf montiert hat, der teilweise aus dem Schlitz (17) herausragt und entlang desselben gleitet, um mindestens einen Abschnitt der Vertiefung des FUDs (20) zu drücken, das über dem Schlitz (17) der Basis (13) angeordnet ist.

8. Vorrichtung (100) nach einem der vorhergehenden Ansprüche, wobei der Ausgabeschlitz (1) der FUD (20) und die horizontale Basis (13) ausgerichtet und beide auf gleicher Höhe angeordnet sind.

9. Vorrichtung (100) nach einem der vorhergehenden Ansprüche, ferner umfassend eine oder mehrere der folgenden Komponenten:
- eine Schalttafel für den Betrieb der elektrischen Komponenten;
- mindestens eine Stromversorgungsplatine (8) zum Steuern und Verwalten der Produktausgabezyklen;
- eine optionale Überwachungskarte zur Verbindung mit einem Touchscreen-Display (2).

## Revendications

1. Distributeur à stérilisation automatique (100) de dispositifs jetables uniques (20) pour aider à la miction des femmes en position debout («Feminine Urinary Device » - FUD), chaque FUD (20) étant fait d'un élément de canal essentiellement conique qui est essentiellement aplati sur les deux génératrices opposées du cône afin d'être distribué et ouvert ensuite de manière tubulaire au moment de l'utilisation,
ledit FUD (20) ayant une base oblique plus large avec un profilé curvilinéaire et doté d'une indentation et une base oblique plus petite, opposée à la base plus large, dotée d'une ouverture formant un bec,
ledit distributeur automatique (100) comprenant
- une pile de dispositifs FUD jetables uniques de forme essentiellement compactée (20) ;
- au moins un conteneur de chargement (9) logeant ladite pile desdits dispositifs FUD jetables uniques de forme essentiellement compactée (20), ledit conteneur (9) reposant sur une base horizontale (13) ;
- une fente de sortie (1) distribuant ledit FUD (20), à proximité de laquelle un ou plusieurs élément(s) de stérilisateur aux UV (12) sont placés, ladite fente (1) étant alignée avec ladite base (13) ;
- un bouton distributeur (3) ;
- au moins un composant électronique (8) pour la commande de la distribution ;
- un moyen de mouvement vers l'avant et de distribution (7) placé en-dessous de ladite base (13) dudit conteneur de chargement (9) et une fente (17) formée à travers ladite base (13),
dans lequel ledit moyen de mouvement vers l'avant (7) est apte à déplacer le FUD (20) reposant sur ladite base (13) vers l'avant sur une certaine longueur en direction de ladite fente de sortie (1), au moyen d'une poussée directe sur ladite indentation dudit FUD (20) de façon à faire sortir de ladite fente de sortie (1) une certaine longueur de FUD à prendre par l'utilisateur,
ledit moyen de mouvement vers l'avant (7) comprenant un élément qui agit en tant que contraste mécanique (16) se déplaçant le long de ladite fente horizontale (17) et ayant une partie faisant saillie vers le haut depuis ladite fente (17), ladite partie en saillie ayant un profilé incurvé, sur le côté frontal vers la fente de sortie (1), qui est complémentaire du profilé de l'indentation du FUD de façon à exercer une poussée directe sur au moins une partie incurvée de ladite indentation dudit FUD (20) lorsque placé au-dessus de ladite fente (17) de ladite base (13).

2. Dispositif (100) selon la revendication 1, doté en outre d'un système de paiement par pièces ou d'autres systèmes de paiement, comme des cartes RFID, des lecteurs de billets et similaire.

3. Dispositif (100) selon la revendication 2, doté en outre d'un écran (2), pouvant être du type écran tactile, pour afficher le coût et/ou les instructions d'utilisation pour l'utilisateur et/ou les opérations guidant l'utilisateur pendant l'achat/le retrait du produit.

4. Dispositif (100) selon l'une quelconque des revendications précédentes, dans lequel ledit conteneur (9) fournit un capteur pour indiquer un produit en rupture de stock ou un stock minimum, connecté à un voyant d'avertissement sur le côté extérieur dudit dispositif.

5. Dispositif (100) selon l'une quelconque des revendications précédentes, dans lequel ledit conteneur (9) est doté en outre d'un élément d'ouverture/de fermeture (10) pour le chargement par l'avant de ladite pile de FUD (20) et/ou d'un contre-poids à forme pour maintenir compactée ladite pile de FUD (20) empilés.

6. Dispositif (100) selon l'une quelconque des revendications précédentes, dans lequel ledit conteneur (9) fournit une ou plusieurs lampe(s) UV (12') de façon à stériliser le FUD (20) se déplaçant vers l'avant en direction de la fente de sortie (1) pendant la distribution.

7. Dispositif (100) selon l'une quelconque des revendications précédentes, dans lequel ledit moyen (7) de mouvement vers l'avant par poussée directe est sous la forme d'un actionneur électromécanique linéaire comprenant :
- un moteur d'entraînement avec une unité de réduction, et
- une tige d'actionneur (14) ayant monté sur son extrémité libre, un bras (15) qui, en retour, a, monté sur son extrémité libre, ledit élément de contraste mécanique (16) avec la tête faisant partiellement saillie de ladite fente (17) et coulissant le long de celle-ci de façon à pousser au moins une partie de ladite indentation dudit FUD (20) qui est placée au-dessus de ladite fente (17) de ladite base (13).

8. Dispositif (100) selon l'une quelconque des revendications précédentes, dans lequel ladite fente de sortie (1) dudit FUD (20) et ladite base horizontale (13) sont toutes deux alignées et placées à la même hauteur.

9. Dispositif (100) selon l'une quelconque des revendications précédentes, comprenant en outre un ou plusieurs des composants suivants :
- un panneau électrique pour le fonctionnement des composants électriques ;
- au moins un circuit électrique (8) pour commander et gérer les cycles de distribution du produit ;
- une carte de surveillance en option pour faire l'interface avec un écran tactile (2).
